(19) Europäisches Patentamt
European Patent Office
Office européen des brevets

(11) Publication number: 0 167 286
A1

(12) # EUROPEAN PATENT APPLICATION

(21) Application number: 85303892.5

(22) Date of filing: 03.06.85

(51) Int. Cl.[4]: C 07 C 49/825
C 07 C 45/46

(30) Priority: 04.06.84 US 616989
21.03.85 US 714407
26.03.85 US 716016
08.04.85 US 721007

(43) Date of publication of application:
08.01.86 Bulletin 86/2

(84) Designated Contracting States:
AT BE CH DE FR GB IT LI LU NL SE

(71) Applicant: CELANESE CORPORATION
1211 Avenue of the Americas
New York New York 10036(US)

(72) Inventor: Davenport, Kenneth Gerald
3125 Crest Water Street
Corpus Christi Texas(US)

(72) Inventor: Hilton, Charles Bruce
505 Williamson Place
Corpus Christi Texas(US)

(72) Inventor: Mott, Graham Nigel
5309 Wentworth Drive
Corpus Christi Texas(US)

(72) Inventor: Keene, Donna Lee
325 Atlantic
Corpus Christi Texas(US)

(74) Representative: De Minvielle-Devaux, Ian Benedict Peter et al,
CARPMAELS & RANSFORD 43, Bloomsbury Square
London WC1A 2RA(GB)

(54) **Process for producing 4-hydroxyacetophenone.**

(57) A process of acetylating phenol to 4-hydroxyacetophenone by contacting phenol with acetic acid or acetic anhydride. When acetic acid is the acetylating agent, per mole of phenol, about 0.9 to 1.4 moles of acetic acid is used in the presence of about 20 to 50 moles of hydrogen fluoride, at a temperature of reaction of about 40 to 90°C, for a reaction period of about 10 to 120 minutes. When acetic anhydride is the acetylating agent, in one aspect of the invention, per mole of phenol, about 0.9 to 2.0 moles of acetic anhydride is used, in the presence of about 8 to 60 moles of hydrogen fluoride, at a temperature of reaction of about 30 to 95°C, for a reaction period of at least about 10 minutes; said temperature of reaction being at least about 45°C when said number of moles of hydrogen fluoride per mole of phenol is no greater than about 12. In another aspect of the invention, about 0.4 to 0.8 moles of acetic anhydride per mole of phenol is used, in the presence of hydrogen fluoride. In all instances, the invention results in phenol conversions of at least about 80% and reaction selectivities to 4-hydroxyacetophenone of at least about 70%.

# PROCESS FOR PRODUCING 4-HYDROXYACETOPHENONE

This invention relates to a process for producing 4-hydroxyacetophenone.

4-Hydroxyacetophenone (4-HAP) is a possible intermediate for a variety of products having a multiplicity of end uses. 4-HAP may be used to make N-acetyl-para-aminophenol (APAP) better known as acetaminophen, which has wide use as an analgesic, or to make 4-acetoxyacetanilide (4-AAA) which can be used for the preparation of poly(ester-amide)s capable of forming an anisotropic melt phase and suitable for being formed into shaped articles such as moldings, fibers and films. In addition, 4-AAA may also be hydrolyzed to form APAP.

Dann and Mylius in a dissertation included as part of a series of Reports from the Institute for Applied Chemistry of the University of Erlangen, received for publication on January 7, 1954 and published in Annalen der Chemie 587 Bank, pages 1 to 15, disclose the reaction of phenol and glacial acetic acid in the presence of hydrogen fluoride to produce 4-hydroxyacetophenone (4-HAP) in a yield of 61.6%. This reaction may be conventionally characterized as a Friedel-Crafts acetylation of phenol with acetic acid as the acetylating agent.

Simons et al, Journal of the American Chemical Society, 61, 1795 and 1796 (1939) teach the acylation of aromatic compounds using hydrogen fluoride as a condensing agent and in Table 1 on page 1796 show the acetylation of phenol with acetic acid to produce p-hydroxyacetophenone (4-HAP) in 40% yield.

European Patent Publication No. 69,597, published January 12, 1983, discloses the preparation of p-phenoxybenzoyl compounds by reacting diphenyl ether and an appropriate acyl compound such as acetic anhydride in the presence of hydrogen fluoride.

Meussdoerffer et al, German Offenlegungsschrift 26 16 986 published October 27, 1977 and assigned to Bayer AG, disclose the hydrogen fluoride-catalyzed acylation of phenolic compounds such as phenol itself with an acyl halide such as acetyl chloride to form hydroxy aromatic ketones.

## SUMMARY OF THE INVENTION

In accordance with this invention, 4-hydroxyacetophenone (4-HAP) is produced by the Friedel-Crafts acetylation of phenol with acetic acid or acetic anhydride using hydrogen fluoride as catalyst, under certain specified reaction conditions so as to obtain unexpectedly high conversions of phenol and high selectivity to 4-HAP, concurrently.

In one embodiment of the present invention, the preparation of 4-HAP by the acetylation of phenol with acetic acid using hydrogen fluoride as catalyst proceeds in accordance with the following equation (I):

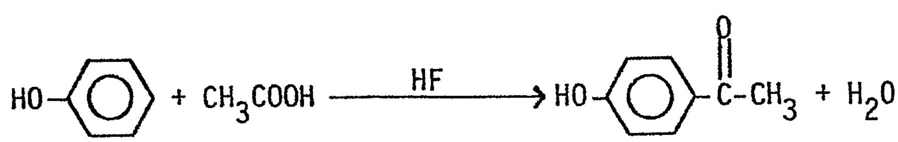


(I)

In carrying out the reaction so as to obtain particularly high yields of 4-HAP within this invention, phenol is reacted with about 0.9 to 1.4 moles, preferably about 1.0 to 1.25 moles of acetic acid per mole of phenol at a temperature of reaction of about 40 to 90$^{\circ}$C, preferably about 50 to 80$^{\circ}$C in the presence of hydrogen fluoride catalyst in an amount of about 20 to 50 moles, preferably about 25 to 30 moles, per mole of phenol for a reaction period of about 10 to 120 minutes, preferably about 30 to 75 minutes.

In another embodiment of the present invention when 4-HAP is prepared from phenol and acetic anhydride, the amount of acetic anhydride

employed may range from about 0.4 to about 2.0 moles per mole of phenol. When about 0.9 to 2.0 moles of acetic anhydride per mole of phenol is employed, using HF as the catalyst, the reaction proceeds in accordance with the following equation (II):

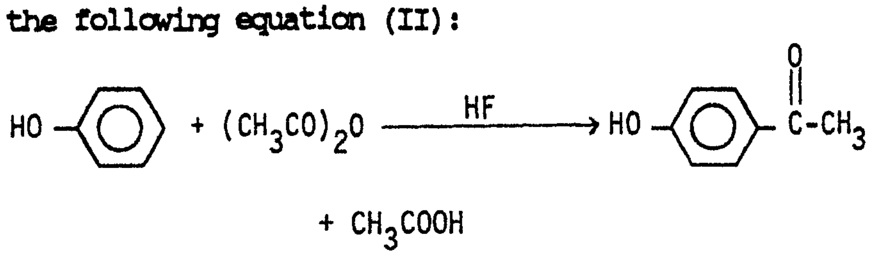


(II)

Thus, in carrying out the reaction so as to obtain particularly high yields of 4-HAP within one aspect of this embodiment of the invention, phenol is reacted with about 0.9 to 2.0 moles, preferably about 1.0 to 1.1 moles of acetic anhydride per mole of phenol at a temperature of reaction of about 30 to 90$^{o}$C, preferably about 40 to 80$^{o}$C in the presence of hydrogen fluoride catalyst in an amount of about 8 to 60 moles, preferably about 15 to 40 moles per mole of phenol for a reaction period of at least about 10, preferably about 10 to 300 minutes, and most preferably about 30 to 180 minutes, said temperature of reaction being at least about 45$^{o}$C when said number of moles of hydrogen fluoride per mole of phenol is no greater than about 12.

In accordance with another aspect, also using acetic anhydride as the acetylating agent, the amount of acetic anhydride employed is approximately one-half mole per mole phenol, such as from about 0.4 to about 0.8 moles of acetic anhydride per mole of phenol.

In such an instance, the anhydride initially added reacts with an equivalent of phenol reactant to produce an equivalent of 4-HAP, yielding a corresponding equivalent of free acetic acid as a by-product. Additional phenol reactant then reacts with the liberated acetic acid in the same reactor to produce an additional amount of 4-HAP. If one half mole of anhydride per mole of phenol is used, the result will be a balanced process with a theoretical yield of one mole of 4-HAP per mole of phenol reactant. If a somewhat larger amount of anhydride is used than one half mole per mole of phenol reactant, an excess of free acid will be produced, while if somewhat less anhydride is used, not all the

phenol reactant will be used up in the reaction, i.e., some phenol reactant will remain at the end of the reaction.

The process employing about one-half mole of acetic anhydride per mole of phenol proceeds as follows in accordance with equations (III) and (IV):

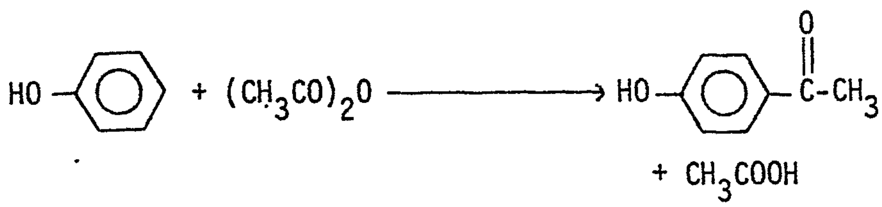

(III)

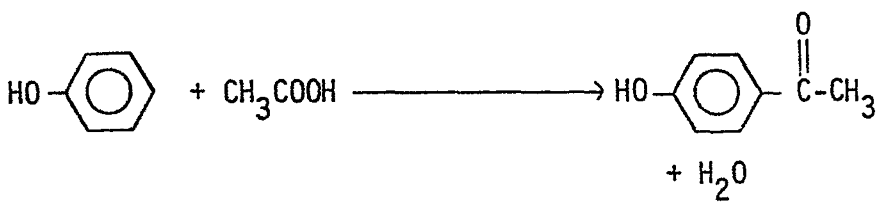

(IV)

When one half mole of acetic anhydride is employed per mole of phenol reactant, the reactions of the overall process may be described as in equation (V):

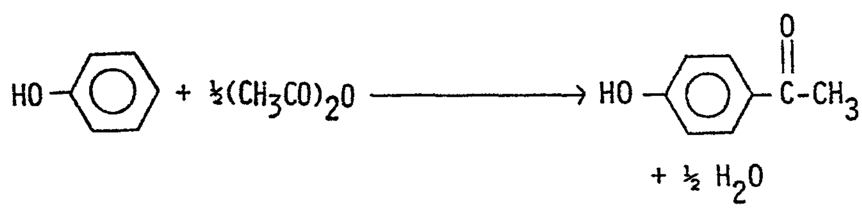

(V)

As stated above, in accordance with the foregoing aspect of the present invention, the phenol reactant is reacted with about 0.4 to 0.8 moles, preferably about 0.45 to 0.55 moles and most preferably about 0.5 mole, of acetic anhydride per mole of phenol at a temperature of reaction, for example, of about 0 to 100°C, in the presence of HF for a reaction period, for example, of about 15 to 120 minutes, preferably about 30 to 75 minutes. An excess of HF is generally used, for example, about 8 to 60 moles per mole of phenol reactant initially present in the reaction zone. In general, the temperature of reaction will be in the range, for example, of about 30 to 90°C, preferably about 40 to 80°C.

In accordance with all embodiments and aspects of the present invention, using a corrosion-resistant reactor, the reaction is initiated by either: 1) charging hydrogen fluoride to a mixture of phenol and acetic acid/anhydride at a temperature less than the specified reaction temperature; 2) charging acetic acid/anhydride to a solution of phenol and hydrogen fluoride at reaction temperature; or 3) charging acetic acid/anhydride and phenol simultaneously to hydrogen fluoride at reaction temperature. Regardless of the method of initially mixing the acetic acid/anhydride, phenol, and hydrogen fluoride, the reaction is then adjusted to the specified reaction temperature for the specified reaction period. The hydrogen fluoride may be charged as a liquid or a gas using technologies of handling well known to those skilled in the art. In carrying out the reaction, an inert gas such as nitrogen may be used to keep the reaction space under the desired pressure, about 2.5 to about 500 psig. (0.17 to 34.48 bar), thereby keeping sufficient HF in contact with the reacting liquid.

The embodiment of this invention using acetic acid as the acetylating agent results in a conversion of phenol of at least about 80%, preferably at least about 90%, with a selectivity to 4-hydroxyacetophenone (4-HAP) of at least about 70%, preferably at least about 80%.

In general, the embodiment of this invention using about 0.9 to 2.0 moles of acetic anhydride per mole of phenol results in a conversion of phenol of at least about 90%, preferably at least about 95%, with a selectivity to 4-hydroxyacetophenone (4-HAP) of at least about 70%, preferably at least about 90%.

When about 0.4 to 0.8 moles of acetic anhydride per mole of phenol is used as the acetylating agent, the process results in a conversion of phenol of at least about 80%, preferably at least about 90%, with a selectivity to 4-hydroxyacetophenone (4-HAP) of at least 70%, preferably at least about 90%.

The aspect of this invention using about 0.5 mole of acetic anhydride per mole of phenol may result in economy of production as compared to the process utilizing on the order of one mole of anhydride or free

acetic acid per mole of phenol reactant as acetylating agent since a considerably smaller quantity of anhydride is employed in this process than the quantity of anhydride or acid in the other processes. Moreover product purification is simpler using such an aspect of this invention since considerably less than one mole of water per mole of phenol reactant need be removed from the product whereas close to one mole of acetic acid or water must be removed when the process utilizes about one mole of anhydride or acid per mole of phenol reactant. Such simpler purification also results in less HF loss.

The present invention is capable of producing relatively high yields of 4-hydroxyacetophenone using relatively low ratios of hydrogen fluoride to phenol and low temperatures of reaction. This in turn results in a more economical purification of product and recycle of hydrogen fluoride, and an energy saving due to the low reaction temperatures which are employed.

DESCRIPTION OF SPECIFIC EMBODIMENTS

The following examples illustrate the process of this invention. The term "molar equivalent" as used in these examples denotes moles of the specified reactant per mole of phenol.

The following examples 1 through 15 relate to the embodiment of the present invention which employs from about 0.9 to 2.0 moles of acetic anhydride per mole of phenol as the acetylating agent.

Example 1

A 300 cc Hastelloy C autoclave was charged with 27.6 g (0.3 mol) of phenol, cooled to -30°C using a carbon dioxide/isopropanol bath, and then evacuated to ca. 175 Torr (0.23 bar). To the autoclave was added 60 g (3.0 mol) of anhydrous hydrogen fluoride during which time an exotherm was noted. The contents of the autoclave were again allowed to cool to -30°C whereupon 30.6 g (0.3 mol) of acetic anhydride was added over a 3 minute period during which time another exotherm was observed. The mixture was warmed to 60°C and maintained at the temperature for 180

minutes with stirring. The hydrogen fluoride was then vented through a caustic scrubber while simultaneously using a nitrogen purge. The contents of the autoclave were then poured onto ice and the pH of the resulting aqueous phase was adjusted to 6.0 using a solution of 45% potassium hydroxide. The aqueous phase was extracted with 75 mL of ethyl acetate (3X). The organic extracts were combined, dried over anhydrous magnesium sulfate, and the solvent was removed on a rotary evaporator to yield a crystalline product. The reaction proceeded with 97.6% conversion of phenol and with the indicated selectivities to phenyl acetate (0.6%), 4-hydroxyacetophenone (79.6%), 2-hydroxyacetophenone (6.2%), and 4-acetoxyacetophenone (1.3%).

Example 2

The procedure of Example 1 was repeated, but employing a reaction time of 270 minutes. The reaction proceeded with 99.0% conversion of phenol and with the indicated selectivities to phenyl acetate (0.2%), 4-hydroxyacetophenone (89.2%), 2-hydroxyacetophenone (4.8%), and 4-acetoxyacetophenone (0.7%).

Example 3

The procedure of Example 1 was repeated, but employing 40 molar equivalents of anhydrous hydrogen fluoride, a reaction temperature of 75°C and a reaction time of 60 minutes. The reaction proceeded with 99.6% conversion of phenol and with the indicated selectivities to phenyl acetate (0.1%), 4-hydroxyacetophenone (84.1%), 2-hydroxyacetophenone (4.7%), and 4-acetoxyacetophenone (0.4%).

Example 4

The procedure of Example 1 was repeated except that the autoclave was initially charged with 18.8 g (0.2 mol) of phenol and 20.4 g (0.2 mol) of acetic anhydride, cooled to -30°C, and evacuated to ca. 175 Torr (0.23 bar), 80 g (4.0 mol) of anhydrous hydrogen fluoride was added, and the mixture was warmed to 75°C and maintained at that temperature for 60 minutes without cooling it a second time to -30°C as described in

Example 1. The hydrogen fluoride was then vented and the remaining procedure of Example 1 followed to recover the product. The reaction proceeded with a 99.7% conversion of phenol and with the indicated selectivities to phenyl acetate (0.1%), 4-hydroxyacetophenone (92.3%), 2-hydroxyacetophenone (5.5%), and 4-acetoxyacetophenone (0.6%).

Example 5

A 300 cc Hastelloy C autoclave was charged with 27.6 g (0.3 mol) of phenol, cooled to -30°C using a carbon dioxide/isopropanol bath, and then evacuated to ca. 175 Torr (0.23 bar). To the autoclave was added 120 g (6.0 mol) of anhydrous hydrogen fluoride during which time an exotherm was noted. The contents of the autoclave were heated to 50°C whereupon 30.6 g (0.3 mol) of acetic anhydride was added over a 3 minute period. An exotherm resulted; the internal temperature of the autoclave was lowered to 50°C and was maintained at that temperature for 120 minutes with stirring. The hydrogen fluoride was then vented through a caustic scrubber while simultaneously using a nitrogen purge. The contents of the autoclave were then poured over ice and the pH of the resulting aqueous phase was adjusted to 6.0 using a solution of 45% potassium hydroxide. The aqueous phase was extracted with 75 mL of methyl acetate (3x). The organic extracts were combined, dried over anhydrous magnesium sulfate, and the solvent was removed on a rotary evaporator to yield a crystalline product. The reaction proceeded with 99.6% conversion of phenol and with the indicated selectivities to phenyl acetate (0.0%), 4-hydroxyacetophenone (77.5%), 2-hydroxyacetophenone (7.2%), and 4-acetoxyacetophenone (0.3%).

Example 6

The procedure of Example 5 was repeated, but employing 1.5 molar equivalents of acetic anhydride. The reaction proceeded with 99.9% conversion and with the indicated selectivities to phenyl acetate (0.1%), 4-hydroxyacetophenone (84.8%), 2-hydroxyacetophenone (7.2%), and 4-acetoxyacetophenone (1.9%).

Example 7

The procedure of Example 5 was repeated, but employing 1.5 molar equivalents of acetic anhydride and a reaction temperature of 75°C. The reaction proceeded with 99.4% conversion and with the indicated selectivities to phenyl acetate (0.1%), 4-hydroxyacetophenone (79.3%), 2-hydroxyacetophenone (8.8%), and 4-acetoxyacetophenone (2.1%).

Example 8

The procedure of Example 5 was repeated, but employing 2.0 molar equivalents of acetic anhydride and a reaction temperature of 75°C. The reaction proceeded with 99.8% conversion and with the indicated selectivities to phenyl acetate (0.3%), 4-hydroxyacetophenone (71.4%), 2-hydroxyacetophenone (8.2%), and 4-acetoxyacetophenone (3.2%).

Example 9

The procedure of Example 5 was repeated, but employing 30 molar equivalents of anhydrous hydrogen fluoride and 1.5 molar equivalents of acetic anhydride. The reaction proceeded with 99.9% conversion and with the indicated selectivities to phenyl acetate (0.0%), 4-hydroxyacetophenone (86.6%), 2-hydroxyacetophenone (6.3%), and 4-acetoxyacetophenone (1.6%).

Example 10

The procedure of Example 5 was repeated, but employing 30 molar equivalents of anhydrous hydrogen fluoride, 1.5 molar equivalents of acetic anhydride, and a reaction temperature of 75°C. The reaction proceeded with 99.9% conversion and with the indicated selectivities to phenyl acetate (0.1%), 4-hydroxyacetophenone (84.2%), 2-hydroxyacetophenone (8.3%), and 4-acetoxyacetophenone (1.3%).

Example 11

A 300 cc Hastelloy C autoclave was cooled to -30°C using a carbon dioxide/isopropanol bath, and then evacuated to ca 175 Torr (0.23 bar).

To the autoclave was added 120 g (6.0 mol) of anhydrous hydrogen fluoride. The contents of the autoclave were heated to 75°C whereupon 27.6 g (0.3 mol) of phenol and 30.6 g (0.3 mol) of acetic anhydride were simultaneously added over a 3 minute period. An exotherm resulted; the internal temperature of the autoclave was lowered to 75°C and was maintained at that temperature for 100 minutes with stirring. The hydrogen fluoride was then vented through a caustic scrubber while simultaneously using a nitrogen purge. The contents of the autoclave were then poured onto ice and the pH of the resulting aqueous phase was adjusted to 6.0 using a solution of 45% potassium hydroxide. The aqueous phase was extracted with 75 mL of ethyl acetate (3x). The organic extracts were combined, dried over anhydrous magnesium sulfate, and the solvent was removed on a rotary evaporator to yield a crystalline product. The reaction proceeded with 99.6% conversion of phenol and with the indicated selectivities to phenyl acetate (0.0%), 4-hydroxyacetophenone (90.5%), 2-hydroxyacetophenone (7.8%), and 4-acetoxyacetophenone (1.1%).

Example 12

The procedure of Example 11 was repeated, but employing 30 molar equivalents of anhydrous hydrogen fluoride, a reaction temperature of 50°C, and reaction time of 60 minutes. The reaction proceeded with 99.6% conversion of phenol and with the indicated selectivities to phenyl acetate (0.1%), 4-hydroxyacetophenone (91.3%), 2-hydroxyacetophenone (5.2%), and 4-acetoxyacetophenone (1.3%).

Example 13

The procedure of Example 11 was repeated, but employing 30 molar equivalents of anhydrous hydrogen fluoride, a reaction temperature of 50°C, and reaction time of 110 minutes. The reaction proceeded with 99.9% conversion of phenol and with the indicated selectivities to phenyl acetate (0.0%), 4-hydroxyacetophenone (89.7), 2-hydroxyacetophenone (4.9%), and 4-acetoxyacetophenone (0.0%).

Example 14

The procedure of Example 11 was repeated, but employing 30 molar equivalents of anhydrous hydrogen fluoride. The reaction proceeded with 99.7% conversion of phenol and with the indicated selectivities to phenyl acetate (0.0%), 4-hydroxyacetophenone (91.4%), 2-hydroxyacetophenone (7.2%), and 4-acetoxyacetophenone (1.1%).

Example 15

The procedure of Example 11 was repeated, but employing 30 molar equivalents of anhydrous hydrogen fluoride, a reaction temperature of 100°C, and reaction time of 10 minutes. The reaction proceeded with 93.4% conversion of phenol and with the indicated selectivities to phenyl acetate (2.5%), 4-hydroxyacetophenone (74.6%), 2-hydroxyacetophenone (13.5%), and 4-acetoxyacetophenone (0.0%).

The following comparative Examples A to E utilize at least one process variable outside the scope of this invention. The results obtained in terms of the selectivity of the reaction for 4-HAP and in most cases phenol conversion, illustrate the significance of carrying out the process using conditions within the ranges defined by the invention.

Comparative Example A

The procedure of Example 1 was repeated, but employing 5 molar equivalents of anhydrous hydrogen fluoride and a reaction temperature of 25°C. The reaction proceeded with 83.5% conversion of phenol and with the indicated selectivities of phenyl acetate (76.0%), 4-hydroxyacetophenone (6.1%), 2-hydroxyacetophenone (0.4%), and 4-acetoxyacetophenone (1.9%).

Comparative Example B

The procedure of Example 1 was repeated, but employing 5 molar equivalents of anhydrous hydrogen fluoride and a reaction temperature of 75°C. The reaction proceeded with 87.0% conversion of phenol and with the indicated selectivities to phenyl acetate (10.3%), 4-hydroxyacetophenone

(64.5%), 2-hydroxyacetophenone (10.5%), and 4-acetoxyacetophenone (1.8%).

Comparative Example C

The procedure of Example 11 was repeated, but employing 5 molar equivalents of anhydrous hydrogen fluoride and reaction time of 30 minutes. The reaction proceeded with 93.3% conversion of phenol and with the indicated selectivities of phenyl acetate (96.2%) and 4-hydroxyacetophenone (0.3%), 2-hydroxyacetophenone (0.0%), and 4-acetoxyacetophenone (0.0%).

Comparative Example D

The procedure of Example 11 was repeated, but employing 30 molar equivalents of anhydrous hydrogen fluoride, a reaction temperature of 20°C, and reaction time of 110 minutes. The reaction proceeded with 82.0% conversion of phenol and with the indicated selectivities to phenyl acetate (6.3%), 4-hydroxyacetophenone (71.5%), 2-hydroxyacetophenone (3.0%), and 4-acetoxyacetophenone (1.7%).

Comparative Example E

The procedure of Example 11 was repeated, but employing 30 molar equivalents of anhydrous hydrogen fluoride, a reaction temperature of 120°C, and reaction time of 2 minutes. The reaction proceeded with 83.2% conversion of phenol and with the indicated selectivities to phenyl acetate (10.9%), 4-hydroxyacetophenone (55.8%), 2-hydroxyacetophenone (12.8%), and 4-acetoxyacetophenone (1.8%).

The significance of the mole ratio of HF to phenol in the claimed process employing from about 0.9 to 2.0 moles of acetic anhydride per mole of phenol as the acetylating agent is illustrated by comparing the results of Examples 3, 4, 11, and 14 under the invention wherein phenol conversions of over 99% and 4-HAP selectivities of over 84% to over 91% were obtained using HF/phenol mole ratios in the range of 10 to 40, with the results of Comparative Example B wherein a phenol conversion of 87%

and a 4-HAP selectivity of 64.5% were obtained using and HF/phenol mole ratio of 5, the other significant process variables being equal or non-critical.

The significance of the lower limit of the reaction temperature range defined by the invention is illustrated by comparing the results of Examples 13 and 14 under the invention wherein phenol conversions of over 99% and 4-HAP selectivities of 89.7% and 91.4% were obtained using reaction temperatures of 50 to 75°C, with the results of Comparative Example D wherein a phenol conversion of 82.0% and a 4-HAP selectivity of 71.5% were obtained using a reaction temperature of 20°C.

The significance of the upper limit of the reaction temperature range in combination with the lower limit of the range of reaction times defined by the invention is shown by comparing the results of Examples 13 and 14 given previously with those of Comparative Example E wherein a conversion of 83.2% and a 4-HAP selectivity of 55.8% was obtained using a reaction temperature of 120°C and a reaction time of 2 minutes.

Examples 16 through 20 relate to the embodiment of the present invention utilizing acetic acid as the acetylating agent.

## Example 16

A 300 cc Hastelloy C autoclave was cooled to -30°C using a carbon dioxide/isopropanol bath, and then evacuated to ca 175 Torr (0.23 bar). To the autoclave was added 75 g (3.75 mol, 30 molar equivalents) of anhydrous hydrogen fluoride. The contents of the autoclave were heated to 80°C whereupon 11.75 g (0.125 mol) of phenol and 7.5 g (0.125 mol, 1 molar equivalent) of acetic acid were simultaneously added over a 1-2 minute period. An exotherm resulted; the internal temperature of the autoclave was lowered to 80°C and was maintained at that temperature for 60 minutes with stirring. The hydrogen fluoride was then vented through a caustic scrubber while simultaneously using a nitrogen purge. The contents of the autoclave were then poured onto ice and the pH of the resulting aqueous phase was adjusted to 6.0 using a solution of 45% potassium hydroxide. The aqueous phase was extracted with 75 mL of

ethyl acetate (3x). The organic extracts were combined, dried over anhydrous magnesium sulfate, and the solvent was removed on a rotary evaporator to yield a crystalline product. The reaction proceeded with 81.9% conversion of phenol and with the indicated selectivities to phenyl acetate (0.4%), 4-hydroxyacetophenone (83.7%), 2-hydroxyacetophenone (9.4%), and 4-acetoxyacetophenone (0.1%).

Example 17

The procedure of Example 16 was repeated, but employing 50 molar equivalents of anhydrous hydrogen fluoride, and 1.1 molar equivalents of acetic acid. The reaction proceeded with 99.3% conversion of phenol and with the indicated selectivities to 4-hydroxyacetophenone (85.9%), 2-hydroxyacetophenone (7.9%), and 4-acetoxyacetophenone (0.1%).

Example 18

The procedure of Example 16 was repeated, but employing 1.25 molar equivalents of acetic acid and a reaction temperature of 73 $\pm$ 2°C. The reaction proceeded with 99.3% conversion of phenol and with the indicated selectivities to 4-hydroxyacetophenone (80.2%), 2-hydroxyacetophenone (7.1%), and 4-acetoxyacetophenone (0.1%).

Example 19

The procedure of Example 16 was repeated, but employing 1.25 molar equivalents of acetic acid and a reaction temperature of 50 $\pm$ 2°C. The reaction proceeded with 93.8% conversion of phenol and with the indicated selectivities to 4-hydroxyacetophenone (90.3%), 2-hydroxyacetophenone (4.6%), and 4-acetoxyacetophenone (0.1%).

Example 20

The procedure of Example 16 was repeated, but employing 1.1 molar equivalents of acetic acid. The reaction proceeded with 97.1% conversion of phenol and with the indicated selectivities to 4-hydroxyacetophenone (80.3%), and 2-hydroxyacetophenone (8.3%).

The following Example 21 relates to the embodiment of the present invention utilizing from about 0.4 to 0.8 moles of acetic anhydride per mole of phenol.

Example 21

A 300 cc Hastelloy C autoclave was charged with 18.8 g (0.2 mol) of phenol, pressurized with nitrogen to 100 psig. (6.9 bar), cooled to -20°C using a carbon dioxide/isopropanol bath, and then evacuated to ca. 175 Torr (0.23 bar). To the autoclave were added 10.2 g (0.1 mol) of acetic anhydride and the contents of the autoclave were cooled to -25°C whereupon 160 g (8.0 mol) of hydrogen fluoride were added. The mixture was warmed to 80°C and maintained at that temperature for one hour with stirring. The contents of the reactor were cooled to 40°C and the hydrogen fluoride was then vented through a caustic scrubber for one hour while simultaneously using a nitrogen purge. The product of the reaction was extracted with 10 mL ethyl acetate, 50 mL of water were added and the pH of the resulting aqueous phase was adjusted to 7.0 using a solution of 45% potassium hydroxide. The aqueous phase was extracted with 50 mL of ethyl acetate (2x). The organic extracts were combined, rinsed with a saturated sodium chloride solution, and the solvent was removed on a rotary evaporator to yield a crystalline product. The reaction proceeded with 97.5% conversion of phenol and with the indicated selectivities to 4-hydroxyacetophenone (64.7%), 2-hydroxyacetophenone (4.6%), and 3-hydroxyacetophenone (0.1%).

What is claimed is:

1. A process for producing 4-hydroxyacetophenone comprising acetylating phenol

(i) with about 0.9 to 2.0 moles of acetic anhydride per mole of phenol, in the presence of about 8 to 60 moles of hydrogen fluoride per mole of phenol, at a temperature of reaction of about 30 to 95°C, for a reaction period of at least 10 minutes, said temperature of reaction being at least about 45°C when said number of moles of hydrogen fluoride per mole of phenol is no greater than about 12; or

(ii) with about 0.9 to 1.4 moles of acetic acid per mole of phenol in the presence of about 20 to 50 moles of hydrogen fluoride per mole of phenol, at a temperature of reaction of about 40 to 90°C, for a reaction period of about 10 to 120 minutes; or

(iii) with about 0.4 to 0.8 moles of acetic anhydride per mole of phenol in the presence of hydrogen fluoride.

2. The process of claim 1 wherein phenol is acetylated with about 0.9 to 2.0 moles of acetic anhydride per mole for phenol, in the presence of about 8 to 60 moles of hydrogen fluoride per mole of phenol, at a temperature of reaction of about 30 to 95°C, for a reaction period of at least 10 minutes, said temperature of reaction being at least about 45°C when said number of moles of hydrogen fluoride per mole of phenol is no greater than about 12.

3. The process of claim 2 wherein a phenol conversion of at least about 90% and a reaction selectivity to 4-hydroxyacetophenone of at least about 70% are obtained.

4. The process of claim 3 wherein about 1.0 to 1.1 moles of acetic anhydride per mole of phenol are employed.

5. The process of claim 4 wherein about 15 to 40 moles of hydrogen fluoride per mole of phenol are employed.

6. The process of claim 5 wherein said temperature of reaction is about 40 to 80°C and said reaction period is 30 to 300 minutes.

7. The process of claim 6 wherein the phenol conversion is at least about 95% and the selectivity to 4-hydroxyacetophenone is at least about 90%.

8. The process of claim 1 wherein phenol is acetylated with about 0.9 to 1.4 moles of acetic acid per mole of phenol in the presence of about 20 to 50 moles of hydrogen fluoride per mole of phenol, at a temperature of reaction of about 40 to 90°C, for a reaction period of about 10 to 120 minutes.

9. The process of claim 8 wherein a phenol conversion of at least about 80% and a reaction selectivity to 4-hydroxyacetophenone of at least about 70% are obtained.

10. The process of claim 8 wherein about 1.0 to 1.25 moles of acetic acid per mole of phenol are employed.

11. The process of claim 10 wherein about 25 to 35 moles of hydrogen fluoride per mole of phenol are employed.

12. The process of claim 11 wherein said temperature of reaction is about 50 to 80°C and said reaction period is 30 to 75 minutes.

13. The process of claim 12 wherein the phenol conversion is at least about 90% and the selectivity to 4-hydroxyacetophenone is at least about 80%.

14. The process of claim 1 wherein phenol is acetylated with about 0.4 to 0.8 moles of acetic anhydride per mole of phenol in the presence of hydrogen fluoride.

15. The process of claim 14 wherein said process is carried out in the presence of about 8 to 60 moles of hydrogen fluoride per mole of phenol as catalyst, at a temperature of reaction of about 30 to 95°C for a reaction period of about 15 to 120 minutes.

16. The process of claim 15 wherein the phenol conversion is at least about 80% and the reaction selectivity to 4-hydroxyacetophenone is at least about 70%.

17. The process of claim 16 wherein about 0.45 to 0.55 moles of acetic anhydride per mole of phenol are employed.

18. The process of claim 17 wherein about 0.5 mole of acetic anhydride per mole of phenol is employed.

19. The process of claim 17 wherein the phenol conversion is at least about 90% and the selectivity to 4-hydroxyacetophenone is at least about 90%.

European Patent Office

# EUROPEAN SEARCH REPORT

0167286
Application number

EP 85 30 3892

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | FR-A-2 348 181 (BAYER AG)<br>* Pages 3,4; claims 1,2,5 * & DE - A - 2 616 986 (Cat. D)<br>--- | 1 | C 07 C 49/825<br>C 07 C 45/46 |
| Y | FR-A-2 357 517 (ICI)<br>* Claims 1,2 *<br>--- | 1 | |
| D,Y | JOURNAL OF THE AMERICAN CHEMICAL SOCIETY, vol. 61, July 1939, pages 1795-1796; J.H. SIMONS et al.: "Hydrogen fluoride as a condensing agent. VII. The acylation of aromatic compounds"<br>* Page 1796 *<br>--- | 1 | |
| Y | EP-A-0 069 597 (RAYCHEM CORP.)<br>* Claims 1-6 *<br>----- | 1 | TECHNICAL FIELDS SEARCHED (Int. Cl.4)<br>C 07 C 45/00<br>C 07 C 49/00 |

The present search report has been drawn up for all claims

| Place of search | Date of completion of the search | Examiner |
|---|---|---|
| THE HAGUE | 04-10-1985 | BONNEVALLE E.I.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document